Europäisches Patentamt

European Patent Office

Office européen des brevets

(11) Publication number: 0 249 338
A2

# (12) EUROPEAN PATENT APPLICATION

(21) Application number: 87304198.2

(22) Date of filing: 12.05.87

(51) Int. Cl.⁴: A61M 25/00

(30) Priority: 12.06.86 US 873615

(43) Date of publication of application:
16.12.87 Bulletin 87/51

(84) Designated Contracting States:
BE DE ES FR GB IT NL

(71) Applicant: C.R. Bard, Inc.
731 Central Avenue
Murray Hill, New Jersey 07974(US)

(72) Inventor: Spector, Kenneth Alan
100 Indian Head Road
Framingham Massachusetts 01701(US)
Inventor: Herman, Stephen Jack
28 Summer Street
Andover Massachusetts 01810(US)
Inventor: Levendusky, Joseph Anthony
21 High Oaks Path
Groton Massachusetts 01450(US)

(74) Representative: Woodward, John Calvin et al
VENNER SHIPLEY & CO. 368 City Road
London EC1V 2QA(GB)

(54) Retroperfusion catheter.

(57) A retroperfusion catheter includes a catheter body and a retroperfusion balloon at the distal end of the body. The catheter also includes means to monitor the pressure at a location distally of the balloon as well as to infuse liquids and medicants downstream of the balloon rapidly and independently of the retroperfusion procedure. The catheter body also has electrode means proximally of the balloon to sense ECG activity directly within the blood vessel as well as to provide a means by which emergency pacing may be stimulated. The catheter construction includes an inner core formed from a soft flexible tube which is surrounded by an overbraid which lends rigidity and torqability for the catheter, the overbraid being surrounded, in turn, by an outer jacket. The distal region of the catheter is bent to define a curve which facilitates steering of the catheter as it is advanced through the patient's vascular system.

## RETROPERFUSION CATHETER

### FIELD OF THE INVENTION

This invention relates to a catheter for use in retroperfusion systems by which oxygenated blood can be shunted from a patient's artery through the patient's venous system to a region of a muscle such as the heart, to which the normal arterial blood supply is obstructed.

### BACKGROUND OF THE INVENTION

This invention relates to blood retroperfusion systems and, particularly, to an improved catheter for use with such systems. Blood retroperfusion is a technique in which a temporary supply of oxygenated blood is delivered to a muscle under circumstances in which the normal flow of oxygenated blood cannot reach the muscle through the patient's artery. For example, blood retroperfusion is a useful procedure when there is a severe blockage in a coronary artery which prevents proper blood flow and nourishment to a portion of the heart muscle. The retroperfusion technique involves shunting temporarily a supply of arterial blood to the undernourished portion of the heart muscle through the coronary vein which leads from the threatened portion of the heart muscle. The technique involves connection of an artery with a vein leading from the endangered portion of the muscle. Blood is pumped from the artery into the vein by a pump which may be synchronized with the patient's normal heart rhythm. Each pulse pumps a volume of blood into the vein so that the blood will flow in a retrograde direction, opposite to the normal flow direction in the vein, and may reach the endangered portion of the muscle. Each pulse of the pump pumps blood to the muscle during the diastolic phase of the patient's heart rhythm. During the systolic pulse of the patient's heart rhythm, the retroperfused blood is permitted to reverse its direction and flow through the vein, in a normal flow direction away from the muscle.

The procedure utilizes a type of balloon catheter such as that described in U.S. Patent 4,290,428 to Durand. The Durand patent describes a catheter which is to be inserted into the vein and has a balloon attached to the distal end of the catheter body. The balloon is inflatable and deflatable and is constructed to form a temporary seal, when inflated, between the catheter and the lumen of the vein. The seal prevents blood from draining while the blood is infused during the diastolic pulse. The inflated balloon assures that the maximum volume of oxygenated blood can flow through the vein to the muscle during diastole. The retrperfusion catheter also is constructed so that the balloon seal will collapse after the diastlic pulse to permit the blood to reverse its flow within the vein and drain away from the muscle. The seal should be made and broken in phase with the systolic-diastolic rhythm of the patient's pulse.

The procedure and apparatus which may be used in the procedure is described generally in Farcot, Synchronized Retroperfusion of Coronary Veins for Circulatory Support of Jeopardized Ischemic Myocardium, The American Journal of Cardiology, June 1978, pp. 1191-1201.

The retroperfusion technique is usable in numerous situations in which the supply of oxygenated blood to the heart muscle is obstructed. This may occur in any arterial location in the body although it presents a particularly critical situation when the obstruction is in a coronary artery which supplies oxygenated blood to the heart muscle itself. Thus, the retroperfusion procedure may be of critical importance in maintaining a supply of oxygenated blood to the heart muscle during those critical moments leading to a potential myocardial infarction (heart attack). The retroperfusion technique, may be administered quickly to maintain alive the threatened portion of the muscle until the patient can be taken to and/or made ready for further treatment.

In addition to its use as an emergency lifesaving technique as described above, the retroperfusion technique also is usable during certain kinds of treatment for arterial obstruction. In recent years another technique known as angioplasty has developed in which a different type of balloon catheter is inserted into the blocked artery and is positioned with its balloon within the narrowed or blocked region. The balloon then is inflated to cause the blocking plaque in the artery to be pressed radially and outwardly against the wall of the artery thereby enlarging the internal bore in the artery. The angioplasty technique has been used in numerous instances as an alternative to more extensive coronary bypass surgery. During the angioplasty procedure, however, the inflation of the angioplasty balloon itself blocks the artery and prevents all flow of blood to the location downstream of the stenosis being treated. Because a complete cutoff of blood flow is itself a threat to the muscle located downstream of the arterial blockage, the angioplasty technique requires that the balloon may be inflated only for relatively short periods of time. By the simultaneous use of retroperfusion, however, oxygenated blood may be supplied to the portions of the muscle which otherwise would be blocked by the angioplasty balloon. Thus, by concurrent use of retroperfusion and angioplasty techniques, the angioplasty procedure may continue for longer periods of time than previously were permitted thereby enabling more complete and better treatment of the stenosis by angioplasty than was previously attainable.

It is important that the retroperfusion procedure be controlled carefully in a number of respects. The pressure developed in the patient's venous system, such as in the coronary sinus, at the distal end of the retroperfusion catheter should not be permitted to become too great. Development of pressure beyond a certain level could cause tissue damage or other difficulties. Additionally, no immediately responsive and effective means is provided by which the pressure within the patient's venous system can be measured quickly and effectively. It is among the general objects of the present invention to provide an improved retroperfusion catheter which enables immediate and direct monitoring of pressure in the venous lumen.

As described, proper functioning of the retroperfusion catheter involves inflating and deflating the balloon synchronously with the patient's systolic-diastolic rhythm. That is accomplished, for example, as described in U.S. Patent No. 4,459,977 to Pizon by sensing the patient's ECG and using the ECG signal to control the rhythm of the catheter. Typically the ECG is detected by leads attached to the patient's skin. Such surface detection of the patient's ECG signal may not be as accurate as might be desired in order to control the retroperfusion system. Surface measurement of a patient's ECG often may include considerable noise and artifact signals from the patient's muscles. Additionally, the effectiveness and accuracy of surface measurement of the patient's ECG is subject to errors resulting from placement of the electrodes on the patient's skin. The ECG signal detected varies at different locations on the patient's skin thus introducing an additional potential variable which could cause difficulty in operating the system.

Retroperfusion techniques may be expected to be used under circumstances where a patient's heart rhythm may become abnormal and the patient may require pacing or immediate infusion of drug or a combination thereof. Should there be a need for immediate temporary pacing, that typically would require insertion of temporary emergency pacing leads. Should the pat.ent require infusion of drugs, administering the drug into the blood which is to be pumped through the lumen of the retroperfusion catheter may not permit a sufficient quantity of drugs to reach the patient in time. The prior retroperfusion catheters are unable to provide a delivery means by whch substantial and effective doses of drugs may be administered directly and immediately to the patient.

It is among the general objects of the invention to provide an improved retroperfusion catheter which overcomes the foregoing difficulties attendant to the retroperfusion technique. The catheter provides a means by which pressure in the coronary sinus may be monitored directly. It provides a means by which drugs and medications may be administered rapidly and directly in sufficient doses as required, the patient's ECG may be detected directly and more accurately to better control the pulsing of the catheter and a means is provided by which a patient's heart rhythm can be be paced immediately, should that be required.

## SUMMARY OF THE INVENTION

The retroperfusion catheter includes a catheter body having a balloon mounted at its distal end. The balloon is shaped to expand into sealed, temporarily obstructing relation with the vein when inflated and to collapse to minimize obstruction to the cross-sectional flow area within the vein when deflated. The body has a main lumen with a distal outlet port at the distal tip of the main body. A plurality of inflation/deflation ports are formed in the catheter body and open into the interior of the balloon. The balloon is inflated and deflated by flow of arterial blood through the inflation/deflation ports. The flow area defined by the inflation/deflation ports is greater than that at the distal outlet port to assure inflation of the balloon into sealed relation with the vein before the arterial blood is ejected from the distal outlet port.

A pair of electrodes is mounted on the catheter proximally of the balloon, and in longitudinally spaced relation. The electrodes are located so that when the balloon is positioned properly the electrodes will be in a position to sense the ECG and/or to apply a pacing pulse to the patient if needed. The electrodes are connected to connectors at the proximal end of the catheter by wires which extend within the wall of the catheter.

The catheter also includes a pressure monitoring lumen which is formed within and extends longitudinally along the wall of the catheter. The lumen has a distal outlet port distally of the balloon, between the balloon and the distal tip of the catheter body. The pressure monitoring lumen connects, at its proximal end, to a tube and a fitting which is connectable to suitable measuring equipment.

The catheter wall body includes a composite structure including an inner tubular core formed from a relatively soft, flexible extruded urethane tube. The main lumen is defined by the inner diameter of the inner tube. The pressure monitoring lumen is formed directly in the wall of the inner tube during the extrusion procedure. The inner tubular core is wrapped with a wire overbraid which provides for stiffness and the assembly is coated with a urethane outer jacket. The wire overbraid terminates short of the balloon so that the most distal end of the catheter remains soft and flexible.

The electrode leads extend along the length of the catheter within the region defined between the inner core and outer jacket. In one embodiment the electrode leads may be formed of insulated wires incorporated into the wire overbraid. Alternately, they may take the form of insulated wires which extend helically along the overbraid. In another embodiment the electrode leads may be contained in channels which are formed as grooves along the outer surface of the inner tube and defined longitudinally extending channels between the outer surface of the inner tube and the wire overbraid.

It is among the general objects of the invention to provide a retroperfusion catheter which enables the patient's distal pressure to be monitored.

Another object of the invention is to provide a retroperfusion catheter which has self-contained means to monitor the patient's cardiac electrical act.vity as well as to provide a readily available means to provide a pacing signal for the patient.

A further object of the invention is to provide a retroperfusion catheter having combined characteristics of flexibility and torsional stiffness to enable the catheter to be torsionally controlled while providing a soft atraumatic distal region for the catheter.

Still another object of the invention is to provide a retroperfusion catheter which is versatile and provides improved means for controlling the patient during the retroperfusion procedure.

## DESCRIPTION OF THE DRAWINGS.

The foregoing and other objects and advantages of the invention will be appreciated more fully from the following further description thereof, with reference to the accompanying drawings wherein:

FIG. 1 is a fragmented illustration of the catheter of the invention with the retroperfus.on balloon illustrated in an inflated configuration;

FIG. 2 is an enlarged side elevation of a partly fragmented section of the distal end of the catheter with the balloon in an inflated configuration;

FIG. 3 is a sectional illustration of the balloon region of the catheter as seen along the line 3-3 of FIG. 2;

FIG. 4 is a sectional illustration similar to FIG. 3 showing the balloon in a collapsed configuration;

FIG. 5 is a fragmented illustration of the main body of the catheter showing the composite structure of the catheter wall and illustrating one embodiment for containing the electrical leads;

FIG. 6 is an illustration of the catheter body having a modified configuration for containing the electrical leads;

FIG. 7 is an illustration of an embodiment of the catheter having still another configuration for containing the electrode leads;

FIG. 8 is a longitudinal fragmented section of the catheter taken along the line 8-8 of FIG. 1 and illustrating the mounting and connection of the electrodes and the catheter structure in the region of the electrodes;

FIG. 9 is a sectional illustration of the catheter taken through the furcated housing;

FIG. 10 is a diagrammatic illustration of the retroperfusion catheter placed alone in the coronary sinus with the balloon in an inflated configuration as when delivering oxygenated blood;

FIG. 11 is a sectional illustration seen along the line 11-11 of FIG. 10 with the balloon collapsed to permit drainage of the blood during systole.

## DESCRIPTION OF THE PREFERRED EMBODIMENT

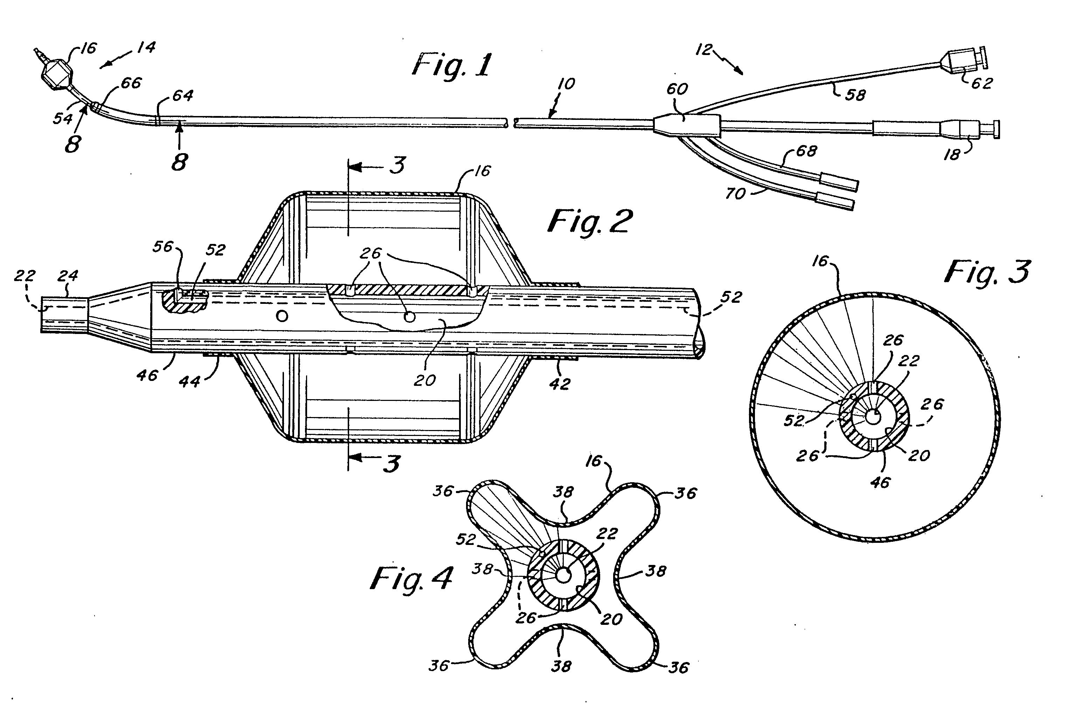

As shown in FIG. 1 the catheter includes an elongated catheter body indicated generally at 10, having a proximal end 12 and a distal end 14. The inflatable and deflatable retroperfusion balloon 16 is mounted to the distal end 14 of the catheter 10. A proximal fitting 18 is mounted to the proximal end 12 of the catheter body 10, the fitting 18 being of luer construction or a similar construction adapted for connection t? a suitable fluid connector. As shown in FIGS. 2 and 3 the catheter body 10 has a main lumen 20 which extends longitudinally of the body and which terminates at a distal outlet port 22 at the distal tip 24 of the catheter body 10. The proximal fitting 18 communicates directly with the proximal end of the main lumen 20. The region of the catheter body 10 which extends through the balloon 16 has a plurality of inflation/deflation ports 26 which communicate between the main lumen 20 and the interior of the balloon 16. The cross-sectional flow area of the inflation-deflation ports 26 is substantially greater that that of the distal outlet port 22 which assures that the balloon will inflate before fluid is ejected from the distal outlet port and also assures that the balloon 16 will collapse quickly when it is intended to be collapsed.

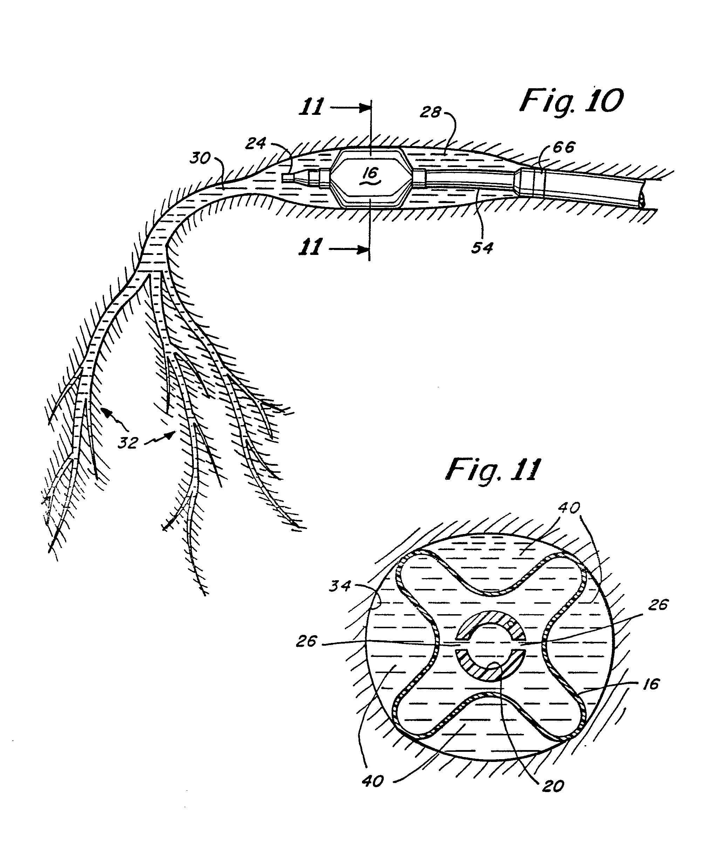

FIGS. 10 and 11 diagrammatically illustrate the placement and operation of the catheter to perform a retroperfusion procedure to direct oxygenated blood to the venous side of patient's heart. As shown, the catheter has been advanced through the superior venae cavae or inferior venae cavae into the right atrium and into the coronary sinus 28. The coronary sinus 28 communicates through the great cardiac vein 30 to smaller branches of the coronary venous tree 32 to the myocardium 33. As illustrated, the catheter is placed so that the balloon 16 is located within the coronary sinus 28. Oxygenated blood is pumped in pulses synchronized with the patient's normal heart rhythm. When a pulse of oxygenated blood is pumped through the catheter, first it fills and inflates the balloon 16 through the larger flow area of the inflation ports 26 to form a temporary seal between the balloon and the lumen 34 of the coronary sinus. The seal prevents blood from draining away from the heart while the oxygenated blood is infused. After the balloon 16 has been inflated, the oxygenated blood is ejected from the smaller flow area of the distal outlet port 22 into the coronary sinus 28 and through the coronary venous tree 32 to the myocardium. During the systolic phase of the patient's heart rhythm the balloon 16 collapses to break its seal within the lumen of the coronary sinus and to permit the retroperfused blood to reverse its direction and flow through the vein, in a normal flow direction, away from the heart.

The balloon 16 is mounted at the distal region of the catheter. It is molded, preferably in a dipping process, from polyvinyl chloride or similar material. The balloon preferably has a uniform wall thickness of about .005 inches and molded in a shape which, when the balloon is relaxed, defines somewhat of a cross or star-shaped in cross-section as shown in FIG. 4. The balloon may be considered as having a cross-sectional shape defined by alternating radially extending lobes 36 and recessed portions 38. The recessed portions 38 will define longitudinal flow passages indicated at 40 in FIG. 11 within the blood vessel 28 to enable retroperfused blood to drain away from the retroperfusion site during systole. When the balloon 16 is expanded under the influence of a pulse of blood, it expands to the circular cross-section shown in FIG. 3 and into sealing contact with the lumen 34 of the blood vessel. The balloon 16 also is formed to include a proximal neck portion 42 and a distal neck portion 44 at the proximal and distal ends of the balloon, respectively, by which the balloon may be attached to the catheter, as will be described in further detail.

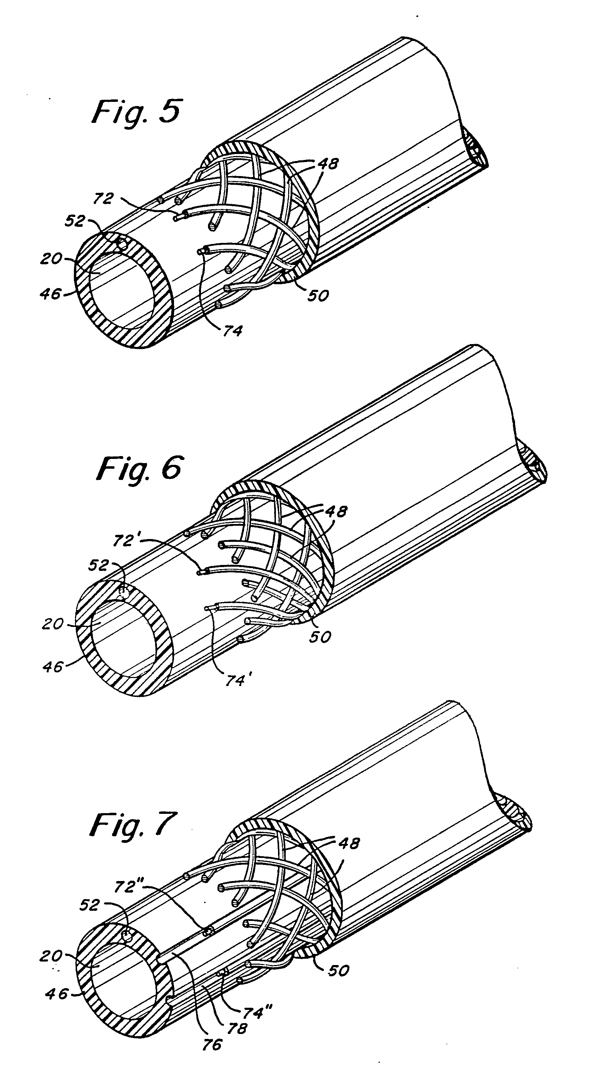

As shown in further detail in FIGS. 5-9 the catheter body 10 is formed from an inner core 46 which is surrounded by a wire overbraid 48 which in turn is covered by an outer jacket 50. The inner core 46 is an extruded tube formed from a soft flexible material, preferably a urethane composition. The inner core 46 extends the full length of the catheter, from the proximal fitting 18 to the distal tip 24. The inner core 46 is extruded to define the main lumen 20 and also to define a smaller secondary lumen 52 which, as will be described, provides pressure measurement and medication infusion capability for the catheter.

In order to provide the catheter with adequate stiffness, a wire overbraid 48 is formed to define a sheath which surrounds the inner core 46. The wire for the overbraid preferably is formed from stainless steel and may have a diameter of the order of .002-.003 inches. The overbraided wire sheath stiffens sufficiently the catheter so that torque may be transmitted to the distal end of the catheter by manipulation of the catheter from its proximal end. It is desirable to be able to transmit torque to the distal end to facilitate guiding of the distal end of the catheter through the patient's venous system to the coronary sinus.

The inner core 46 and wire overbraid 48 are encased in an outer jacket 50. The outer tubular jacket 50 also may be formed from urethane and fits firmly about the core 46 and overbraid 48. The composite inner core 46, wire overbraid 48 and outer jacket 50 extend from the proximal fitting 18 toward the distal tip 24 of the catheter. The wire overbraid 48 and outer jacket 50 terminate short of the distal tip 24 of the catheter so that a distal segment 54 of the inner core 46 protrudes distally beyond the overbraid 48 and outer jacket 50. The distal segment 54 provides a soft, flexible and atraumatic tip which reduces substantially the risk of trauma or injury to the patient.

By way of dimensional example, the inner core may have an outer diameter of the order of .090 inches and a wall thickness of about .018 inches. The outer jacket 50 may have a wall thickness of about .003-.005 inches. The catheter may be made in varying lengths and, for example, may be of between 50 cms. and 100 cms. in length.

In order to facilitate steering of the distal end of the catheter, the distal end is provided with a preset curve or bend as indicated in FIG. 1. The curve may be similar to the curve formed in Cournand catheters. The combination of the curved distal end and the ability of the catheter shaft to transmit torque from the proximal end to the distal end enables the physician to control the drection in which the distal end extends, as it is advanced through the patient's blood vessels.

The balloon 16 is attached to the distal segment 54 by adhesively attaching the neck portions 42, 44 of the balloon to the distal segment 54. The inflation/deflation ports 26 are formed in the distal segment 54 of the inner core 46 to communicate directly with the interior of the balloon 16.

The portion of the distal segment 54 of the inner core 46 which is located beyond the fluid port 56 is tapered to a necked-down configuration having a first tapered portion 53 and a terminal untapered portion 55. The diameter of the main lumen 20 reduces progressively as it passes through the necked-down tip 53, 55 of the catheter to define a relatively small diameter outlet port 22 at the distal tip 24. By way of example, in the preferred embodiment of the invention, the main lumen 20 may have an inner diameter of the order of .055 inches. The diameter of the outlet port 22 may be reduced to an inner diameter of about .045 inches. In the preferred embodiment of the invention six or eight inflation/deflation ports 26 are provided and each may have a diameter of the order of .038 inches. Thus, the flow area defined by the inflation/deflation ports is substantially greater than that at the most distal outlet port 22.

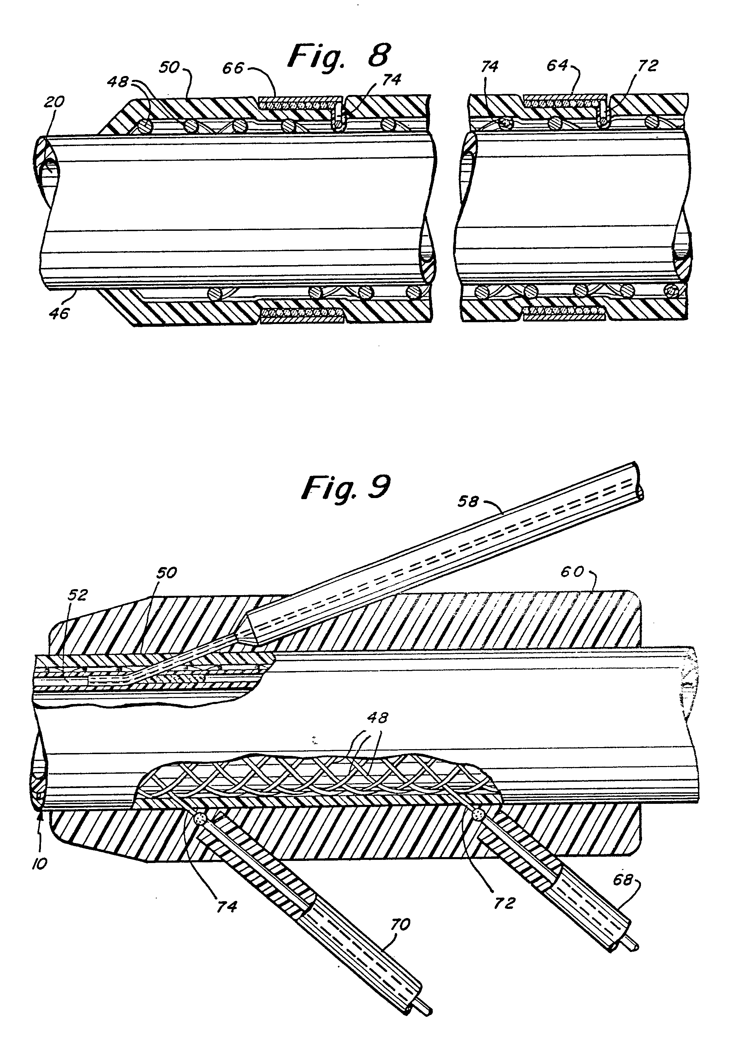

The balloon is located so that its distal neck 44 is spaced proximally from the distal tip 24 of the catheter. The distal end of the inner core 46, which extends distally beyond the distal neck 44 of the balloon 16 is formed to define a fluid port 56 through the wall of the inner core 46. The fluid port 56 communicates with and defines the distal end of the secondary lumen 52, thereby to provide an independent means of fluid communication from the proximal end of the catheter to a location outside of the catheter and distally of the balloon. As shown in FIG. 9 access to the secondary lumen 52 from the proximal end of the catheter is provided by means of tubular side leg 58 which is connected to the proximal regon of the catheter by a furcated housing 60. The lumen 59 of the side leg 58 is connected to the catheter within the furcated housing 60 so as to be in communication with the secondary lumen 52. The distal end of the side leg 58 may be reduced in diameter, as indicated at 57 to enable the reduced diameter portion 57 to be passed through the wall of the inner core 46 and into the secondary lumen 52. The proximal end of the side leg 58 s provided with a fitting 62, such as a Luer-Lok device. The fitting 62 is connectible to a pressure monitoring device by which the pressure downstream of the balloon 16 may be measured. It also may be connected to a source of liquid infusion, for example, to infuse medication directly to the venous site.

The catheter also includes electrode means by which the patient's E.C.G. may be monitored. As shown in FIGS. 1 and 8 the catheter preferably has a pair of ring electrodes 64, 66 which are mounted on the catheter in spaced relation, proximally of the balloon. At least one of the electrodes, distal electrode 66, is placed on the catheter close enough to the balloon so that when the balloon is in place in the coronary sinus, the distal electrode 66 also will be disposed within the coronary sinus 28. For example, in the illustrative embodiment, the distal electrode 66 may be located about 2 to 3 cm. proximally of the balloon 16. The proximal electrode 64 is spaced from the distal electrode and may be located so that it will be disposed in the patient's right atrium or in the coronary sinus. The electrodes 64, 66 may be formed from thin wall (about .002 inches thick) stainless steel rings. The ring electrodes have an outer diameter corresponding to the outer diameter of the outer jacket 50 to present a smooth external configuration.

The electrodes 64, 66 are connectible to external ECG equipment by a pair of electrical leads 68, 70. The leads 68, 70 are secured to the catheter body by the furcated housing 60 which is molded to and about the catheter body 10, the leads 68, 70 and the side leg 58. The electrical leads 68, 70 are electrically connected to the electrodes 64, 66, respectively by wires extending through the catheter. As shown in FIGS. 5 and 8 the wires 72, 74 may be formed integrally with the wire overbraid 48 to serve the dual function of contributing to the overbraid structure as well as providing electrical communication. The wires 72, 74 are independently insulated and preferably the insulation 73 has an outer diameter which is substantially the same as the diameter of the other wires which form the overbraid.

The wires 72, 74 are connected to the electrodes 64, 66 in the manner illustrated in FIG. 8. The distal ends of the wires 72, 74 are exposed by stripping the insulation and the exposed ends are passed through an opening in the wall of the outer jacket 50 and are wound a number of turns about the outer jacket 50. The electrodes 64, 66 overlie the wound ends of the wires 72, 74, respectively. The ends of the wires 72, 74 which are wound about the outer jacket 50 are contained securely by the surrounding ring electrodes 64, 66, respectively. The wall of the outer jacket 50 may be slightly compressed in the region of the electrodes so as to maintain the outer surface of the electrodes 64, 66 substantially flush with the outer surface of the outer jacket 50. A suitable adhesive also may be used to secure the electrodes and wires in place on the catheter, if desired.

FIG. 6 illustrates an alternate arrangement for the wires 72', 74' in which the wires are wound helically about the overbraid without forming a direct part of the overbraid structure itself.

FIG. 7 illustrates still another embodiment in which the wires 72", 74" are contained within longitudinally extending channels 76, 78 formed along the outer surface of the inner core 46. In each case, the wires are individually insulated. The distal end of each of the wires is connected to its associated electrode 64 or 66 as described above.

As mentioned above the distal region of the catheter preferably is formed to define a curved configuration to facilitate guiding of the catheter as it is advanced through the patient's blood vessels. It is preferred, however, that the balloon 16 be mounted on an uncurved, straight portion of the catheter. To that end, the curve is formed in the distal segment 58 of the catheter so that the portion which is to carry the ballon 16 is straight, or at least is provided with a minimal curve.

In use the catheter is placed, as described, with the balloon and at least the distal electrode located in the patient's coronary sinus 28. The fitting 18 is connected to the outlet from a suitable synchronous pumping system which also is connected to the leads 68, 70 so that the system may be operated in synchronization with the patient's heart rhythm. Sensing of the patients E.C.G. activity by direct contact with the coronary sinus provides for more accurate monitoring of the patient's E.C.G. with consequent more accurate synchronization of the retroperfsion pulsing. Additionally, should there be a need for emergency pacing, it is unnecessary to incur the loss of time and inconvenience needed to insert emergency pacing leads. Pacing pulses can be applied directly and immediately to the patient. Moreover, the patient's blood pressure downstream of the balloon may be monitored at all times during the retroperfusion cycle to assure that excessive pressure levels are not developed. In addition to providing a means by which the blood pressure may be measured and monitored, the catheter also provides means by which medication may be delivered directly to the patient's heart independently of the retroperfusion procedure and at any time.

—

## Claims

1. A retroperfusion catheter comprising:
an elongated catheter body having a proximal end and a distal end, the body having a central lumen extending from its proximal end to its distal end, the lumen terminating at its distal end at a distal outlet port;
a ballon mounted to the distal end of the body, the balloon being in communication with the central lumen whereby the balloon may be inflated and deflated in response to changes in pressure within the central lumen; and
means for monitoring the pressure at a location distally of the balloon independently of operation of the balloon.

2. A retroperfusion catheter as defined in claim 1 wherein said means for monitoring pressure comprises:
a secondary lumen formed through and along the catheter body, the secondary lumen having an outlet port distally of the balloon, the secondary lumen having an inlet at the proximal region of the catheter.

3. A retroperfusion catheter as defined in claim 2 further comprising:
the catheter body being formed from an inner elongated tubular core having the main lumen formed therethrough; and
the second lumen being formed through the wall of the core.

4. A retroperfusion catheter as defined in claim 3 further comprising:
a wire overbraid formed about the inner core; and
an outer jacket formed about the wire overbraid.

5. A catheter as defined in claim 4 further comprising:
a furcation formed on the catheter, the furcation having a side leg in communication with the secondary lumen; and
a proximal fitting attached to the proximal end of the catheter body.

6. A catheter as defined in claim 4 further comprising:
the wire overbraid and outer jacket terminating short of the distal end of the tubular core thereby to define a distally protruding segment of the tubular core;
said balloon being mounted on the distal segment.

7. A retroperfusion catheter as defined in claim 6 further comprising:
said inner core being formed from a relatively soft, flexible material whereby the distal segment of the catheter is relatively soft and flexible thereby to present a relatively atraumatic tip as the catheter is advanced in a patient's vascular system.

8. A retroperfusion catheter as defined in claim 6 further comprising:
the distal region of the catheter being curved.

9. A retroperfusion catheter as defined in claim 1 further comprising:

means carried by the catheter body for monitoring electrical activity of the blood vessel in which the balloon is placed.

10. A retroperfusion catheter as defined in claim 9 wherein the electrical means comprises:

a pair of spaced electrodes on the catheter body proximally of the balloon; and

electrical conductor means extending through the catheter body and being connected to the electrodes at their distal ends, the proximal ends of the conductor being connected to leads extending out of the catheter body at the proximal region of the catheter body.

11. A catheter as defined in claim 10 wherein the electrodes are located so that at least one of the electrodes may be located within the coronary sinus when the balloon is in the coronary sinus.

12. A retroperfusion catheter as defined in claim 10 further comprising:

a catheter body being formed from an inner elongated tubular core having the main lumen formed therethrough:

the secondary lumen being formed through the wall of the core;

a wire overbraid formed about the inner core; and

an outer jacket formed about the wire overbraid.

13. A retroperfusion catheter as defined in claim 12 further comprising:

said electrically conductor means being formed integrally with and defining strands of the wire overbraid.

14. A retroperfusion catheter as defined in claim 12 further comprising:

said electrically conductor means being wrapped helically about the wire overbraid.

15. A retroperfusion catheter as defined in claim 12 further comprising:

means defining longitudinally extending grooves in the outer surface of the inner core, said electrical conductor means being disposed within the grooves.

16. A retroperfusion catheter comprising:

an elongated catheter body having a lumen extending therethrough and a retroperfusion balloon at its distal end, the lumen being in communication with the interior of the balloon, the lumen terminating in a distal outlet tip; and

electrode means carried by the catheter body for monitoring electrical activity of the blood vessel in which the balloon is placed.

17. A method for performing retroperfusion comprising:

placing a retroperfusion catheter in a selected location of a patient's venous system and performing a retroperfusion procedure;

selectively performing at least one of the steps of monitoring the pressure in the blood vessel sensing electrical activity of the blood vessel, applying a pacing pulse to the blood vessel and infusion of liquids into the blood vessel independently of the retroperfusion procedure.

18. A method for performing retroperfusion as defined in claim 17 further comprising:

performing at least said steps of monitoring the pressure in the blood vessel and sensing electrical activity of the blood vessel.

Fig.1

Fig.2

Fig.3

Fig.4

*Fig. 5*

*Fig. 6*

*Fig. 7*

*Fig. 8*

*Fig. 9*

*Fig. 10*

*Fig. 11*